# EUROPÄISCHE PATENTSCHRIFT

(11) **EP 0 420 794 B1**
(45) Veröffentlichungstag und Bekanntmachung des Hinweises auf die Patenterteilung: **20.10.1993**
(21) Anmeldenummer: 90810630.5
(22) Anmeldetag: 21.08.1990
(51) Int. Cl.: A61F 2/42

(54) **Fingergelenkprothese**
Finger joint prosthesis
Prothèse pour articulation du doigt

(30) Priorität: 28.09.1989 CH 3525/89
(43) Veröffentlichungstag der Anmeldung: 03.04.1991
(73) Patentinhaber: SULZER Medizinaltechnik AG, 8404 Winterthur (CH); PROTEK AG, 3110 Münsingen-Bern (CH)
(72) Erfinder: Meuli, Hans-Christoph, Prof. Dr.-med., CH-3012 Bern (CH); Frey, Otto, Dr., CH-8400 Winterthur (CH)
(74) Vertreter: Hammer, Bruno, Dr.

(56) Entgegenhaltungen:
- EP-A- 0 176 711
- FR-A- 2 605 878
- US-A- 4 231 121
- US-A- 4 685 919

## Beschreibung

Die Erfindung betrifft eine Fingergelenkprothese, die aus zwei aufeinander gleitenden Prothesenhälften besteht, die jeweils in Form von sich verjüngenden Zapfen ausgebildet und im Knochen verankerbar sind.

Fingergelenkprothesen sind in der CH-PS 542 624 und in der DE-PS 23 38 136 / DE-PS 23 38 137 beschrieben. In beiden Fällen handelt es sich um Scharnierbauweise mit mechanischer Erstverankerung durch Aufspreizen eines Dorns oder einer Hülse in verkürzten und vorgebohrten Knochengliedern.

Die gattungsbildende französische Patentanmeldung FR-A-2 605 878 zeigt ebenfalls eine Fingergelenkprothese mit zwei Prothesenhälften, die als konische Zapfen eine Primärverankerung ohne Knochenzement erfahren. Die Prothesenhälften bestehen aus festem mechanisch bearbeitbarem Material derart, dass der proximale Prothesenteil mindestens an der Gleitfläche in Metall ausgeführt ist, während der distale Prothesenteil mindestens an der Gleitfläche aus einem thermoplastischen Material ausgeführt ist. Eine derartige Verankerung setzt voraus, dass die für die Aufnahme der Prothesenzapfen vorgesehenen Knochen eine für eine Primärverankerung notwendige Festigkeit aufweisen und dass sekundär ein Einwachsen von Knochenmaterial stattfindet.

Fingergelenkprothesen sind relativ selten, da die Fingerchirurgie mit körpereigenem Material einen hohen Stand erreicht hat. Fingergelenkprothesen kommen vor allem bei Gelenkveränderungen infolge chronischer Polyarthritis und Arthrose in Frage. Ausgerechnet in diesen Fällen ist aber meist eine hochgradige Zerstörung auch des Bandapparates und des Gelenkkapselapparates vorhanden. Ein künstliches Fingergelenk hat deshalb in erster Linie Platzhalterfunktion und muss eine ausreichende seitliche Primärstabilität garantieren.

Hier schafft die Erfindung Abhilfe. Sie löst die Aufgabe, dem Gelenk einen Teil seiner Funktion zurückzugeben.

Gemäss der Erfindung wird die Aufgabe gelöst, indem die Prothesenhälften aus einem Sintergefüge von Hydroxyl-Apatit bestehen und indem diese Zapfen durch eine gleitfähige und den Knochenaufbau hemmende Zwischenlage voneinander getrennt sind.

Die Vorteile der Erfindung sind darin zu sehen, dass das Implantat selbst bis auf eine Zwischenschicht aus einem Material besteht, das die Knochenbildung fördert, ohne sich selbst zu schnell abzubauen, dass die Knochenbildung durch eine ständige, nicht zu grosse Belastung unterstützt wird, und dass damit eine Altersgruppe, die vorher davon ausgeschlossen war, die Möglichkeit erhält, die Teilfunktion von Fingergelenken zurückzuerlangen.

Im folgenden wird die Erfindung anhand eines Ausführungsbeispiels beschrieben. Es zeigen:

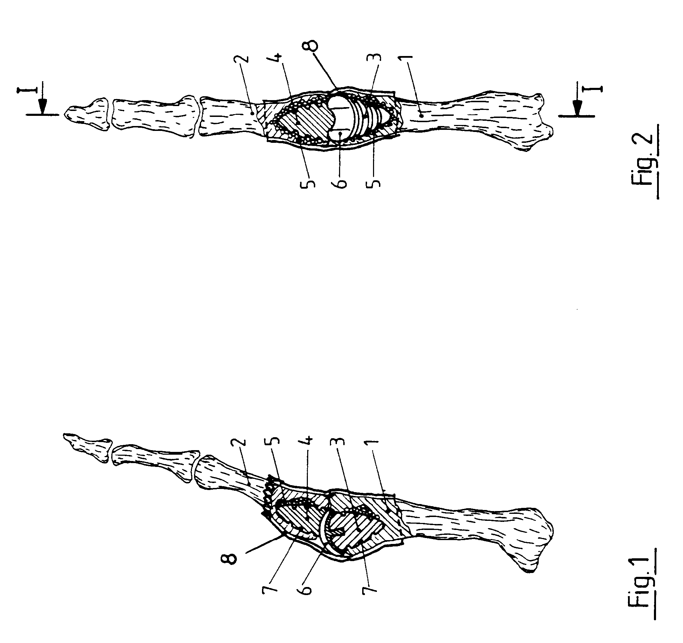

- Fig. 1: die Seitenansicht eines Schnittes durch die Knochen eines Fingers mit eingesetzter Prothese und
- Fig. 2: die Draufsicht auf die Knochen eines Fingers mit eingesetzter Prothese entsprechend Fig. 1.

In den Figuren ist eine Fingergelenkprothese gezeigt, die aus zwei aufeinander gleitenden Prothesenhälften besteht, die jeweils in einem der dem Gelenk zugeordneten Knochenenden 1, 2 verankerbar sind.

Erfindungsgemäss bestehen die Prothesenhälften aus einem Sintergefüge von Hydroxyl-Apatit, das in Form von sich verjüngenden Zapfen 3, 4 im Knochen 1, 2 verankert ist, wobei die Zapfen 3, 4 durch eine gleitfähige und den Knochenaufbau hemmende Zwischenlage 6 aus Polyurethan oder einem anderen, körperverträglichen Kunststoff voneinander getrennt sind. Die Zwischenlage 6 selbst ist auf dem proximalen Zapfen 3 verankert und bildet mit dem auf ihr gleitenden Zapfen 4 aufeinander abgestimmte konkave und konvexe Lagerflächen, die eine geführte Bewegung in der Beuge-Streckebene zulassen.

Zur besseren Verankerung weisen die Zapfen 3, 4 Hinterschneidungen 7 und Rillen auf, die den Halt nach dem Einwachsen des Knochengewebes sicherstellen. Beim Einbringen der Zapfen 3, 4 werden diese mit Granulat 5 aus Hydroxyl-Apatit verkeilt und alle Hohlräume zwischen Zapfen 3, 4 und den zugehörigen Knochenenden 1, 2 werden mit Granulat 5 aus Hydroxyl-Apatit gestopft, wobei das Granulat mit seinem grossen Oberflächenanteil relativ schnell zur Knochenbildung führt. Die Abmessungen des implantierten Gelenkkopfes sind grösser als das ursprüngliche Gelenk gehalten.

Um dem Fingergelenk bis zum Verwachsen der Gelenkkapsel genügend seitliche Stabilität zu geben, kann ein elastischer Strumpf (8) aus resorbierbarem Material wie z.B. Gelatine oder Polylactat über das Gelenk gezogen werden.

## Patentansprüche

1. Fingergelenkprothese bestehend aus zwei aufeinander gleitenden Prothesenhälften, die in Form von sich verjüngenden Zapfen (3, 4) ausgebildet und im Knochen (1, 2) verankerbar sind, dadurch gekennzeichnet, dass die Prothesenhälften aus einem Sintergefüge von Hydroxyl-Apatit bestehen und dass die Zapfen (3, 4) durch eine gleitfähige und den Knochenaufbau hemmende Zwischenlage (6) voneinander getrennt sind.

2. Fingergelenkprothese nach Anspruch 1, dadurch gekennzeichnet, dass die Zapfen (3, 4) Hinterschneidungen (7) und Rillen aufweisen.

3. Fingergelenkprothese nach einem der Ansprüche 1 oder 2, dadurch gekennzeichnet, dass die Zapfen (3, 4) zusammen mit der Zwischenlage (6) aufeinander abgestimmte konkave und konvexe Lagerflächen bilden.

4. Fingergelenkprothese nach einem der Ansprüche 1 bis 3, dadurch gekennzeichnet, dass die Zwischenlage (6) auf einem der Zapfen (3, 4) verankert ist.

5. Fingergelenkprothese nach einem der Ansprüche 1 bis 4, dadurch gekennzeichnet, dass die Gelenkbewegung durch die Lagerflächen von Zwischenlage (6) und Zapfen (3, 4) in einer Ebene geführt ist, solange Haltekräfte das Gelenk zusammenhalten.

6. Fingergelenkprothese nach einem der Ansprüche 1 bis 5, dadurch gekennzeichnet, dass die Gelenkabmessungen der Prothese deutlich grösser sind als die Abmessungen des ursprünglichen Gelenks.

7. Fingergelenkprothese nach einem der Ansprüche 1 bis 6, dadurch gekennzeichnet, dass die Zwischenlage (6) aus Polyurethan oder anderen körperverträglichen Kunststoffen besteht.

8. Fingergelenkprothese nach einem der Ansprüche 1 bis 7, dadurch gekennzeichnet, dass die Prothese von einem schlauchartigen, elastischen Gewebe (8) umhüllt ist, welches einerseits genügend Steifigkeit für das Erreichen einer ausreichenden seitlichen Primärstabilität aufweist und andererseits resorbierbar ist.

## Claims

1. A finger joint prosthesis consisting of
two half-prostheses which slide against one another and are made in the form of tapering pegs (3, 4) which may be anchored in the bones (1, 2), characterized in that the half-prostheses consist of a sintered structure of hydroxyl apatite and that the pegs (3, 4) are separated from one another by an intermediate layer (6) which is capable of sliding and impedes the build-up of bone.

2. A finger joint prosthesis
as in Claim 1, characterized in that
the pegs (3, 4) exhibit undercuts (7) and grooves.

3. A finger joint prosthesis
as in one of the Claims 1 or 2, characterized in that the pegs (3, 4) together with the intermediate layer (6) form concave and convex bearing areas which are matched to one another.

4. A finger Joint prosthesis
as in one of the Claims 1 to 3, characterized in that the intermediate layer (6) is anchored to one of the pegs (3, 4).

5. A finger joint prosthesis
as in one of the Claims 1 to 4, characterized in that the motion of the joint is guided in one plane by the bearing areas of the intermediate layer (6) and the pegs (3, 4), so long as retaining forces hold the joint together.

6. A finger joint prosthesis
as in one of the Claims 1 to 5, characterized in that the dimensions of the joint of the prosthesis are distinctly greater than the dimensions of the original joint.

7. A finger joint prosthesis
as in one of the Claims 1 to 6, characterized in that the intermediate layer (6) consists of polyurethane or other bodily compatible plastics.

8. A finger joint prosthesis
as in one of the Claims 1 to 7, characterized in that the prosthesis is wrapped in a hoselike elastic tissus (8) which on the one hand exhibits enough stiffness to achieve adequate primary lateral stability and on the other hand is resorbable.

## Revendications

1. Prothèse articulaire de doigt constituée de deux moitiés prothétiques glissant l'une sur l'autre, qui sont réalisées en forme de tenons effilés (3, 4) et qui peuvent être ancrées dans l'os (1, 2), caractérisée en ce que les moitiés prothétiques consistent en une structure frittée en hydroxy-apatite et en ce que les tenons (3, 4) sont séparés par une couche intermédiaire (6) apte à glisser et ralentissant l'ostéogenèse.

2. Prothèse articulaire de doigt suivant la revendication 1, caractérisée en ce que les tenons (3, 4) présentent des contre-dépouilles (7) et des rainures.

3. Prothèse articulaire de doigt suivant l'une des revendications 1 ou 2, caractérisée en ce que les tenons (3, 4) forment avec la couche intermédiaire (6) des surfaces d'appui concaves et convexes coordonnées les unes par rapport aux autres.

4. Prothèse articulaire de doigt suivant l'une des revendications 1 à 3, caractérisée en ce que la couche intermédiaire (6) est ancrée sur l'un des tenons (3, 4).

5. Prothèse articulaire de doigt suivant l'une des revendications 1 à 4, caractérisée en ce que le mouvement de l'articulation est guidé dans un même plan par les surfaces d'appui de la couche intermédiaire (6) et des tenons (3, 4), aussi longtemps que des forces de maintien retiennent l'articulation.

6. Prothèse articulaire de doigt suivant l'une des revendications 1 à 5, caractérisée en ce que les dimensions articulaires de la prothèse sont nettement plus élevées que les dimensions de l'articulation initiale.

7. Prothèse articulaire de doigt suivant l'une des revendications 1 à 6, caractérisée en ce que la couche intermédiaire (6) est réalisée en polyuréthanne ou en d'autres matières plastiques tolérées par l'organisme.

8. Prothèse articulaire de doigt suivant l'une des revendications 1 à 7, caractérisée en ce que la prothèse est entourée par un tissu (8) élastique tubulaire, qui d'un côté présente suffisamment de rigidité pour l'obtention d'une stabilité primaire latérale suffisante et qui, d'autre part, est résorbable.
